# EUROPÄISCHE PATENTSCHRIFT

(11) **EP 2 748 593 B1**
(45) Veröffentlichungstag und Bekanntmachung des Hinweises auf die Patenterteilung: **24.05.2017**
(21) Anmeldenummer: 12766905.9
(22) Anmeldetag: 15.08.2012
(51) Int. Cl.: G01N 23/04, G01V 5/00, G01T 7/00, G01N 33/22, G01N 23/02

(54) **FLÜSSIGES GEMISCH ZUM TESTEN UND VALIDIEREN VON PRÜFGERÄTEN**
LIQUID MIXTURE USED TO TEST AND VALIDATE TEST DEVICES
MÉLANGE LIQUIDE POUR TESTER ET VALIDER DES APPAREILS DE CONTRÔLE

(30) Priorität: 22.08.2011 DE 102011081328; 10.11.2011 DE 102011118107
(43) Veröffentlichungstag der Anmeldung: 02.07.2014
(73) Patentinhaber: Smiths Heimann GmbH, 65205 Wiesbaden (DE)
(72) Erfinder: SIEDENBURG, Uwe, 55270 Essenheim (DE)
(74) Vertreter: Greif, Thomas
(86) Internationale Anmeldenummer: PCT/EP2012/065950
(87) Internationale Veröffentlichungsnummer: WO 2013/026753

(56) Entgegenhaltungen:
- GB-A- 347 144

## Beschreibung

In der deutschen Patentanmeldung DE 10 2011 081 328.4 ist beschrieben, zur Überprüfung von Personen und Objekten, wie Gepäckstücken, auf gefährliche Stoffe, wie Spreng- oder Explosivstoffe, Prüfanlagen zu verwenden, in denen die zu prüfenden Personen oder Objekte in Prüfgeräte von elektromagnetischen Strahlen durchleuchtet oder bestrahlt werden. Bekannterweise werden derartige Prüfanlagen an Flughäfen zur Überprüfung von Passagieren und Gepäckstücken eingesetzt.

Die Prüfanlagen enthalten nach einer bekannten Ausführungsform Prüfgeräte, bei denen die zu überprüfenden Objekte, beispielsweise die Gepäckstücke, von Röntgenstrahlen durchleuchtet oder bestrahlt werden und die transmittierten oder gestreuten Röntgenstrahlen detektiert und ausgewertet werden (DE 10125531-A, DE 19954662-A).

Zur Überprüfung von Personen sind Prüfgeräte bekannt, bei denen die zu überprüfenden Personen mit elektromagnetischen mm-Wellen bestrahlt und die gestreuten mm-Wellen für eine bildhafte Darstellung ausgewertet werden (DE 102005016106-A).

Vor der Inbetriebnahme müssen die Prüfgeräte getestet und validiert werden. Dies erfolgt üblicherweise mit den realen Gefahrstoffen, also den zu detektierenden Sprengstoffen. Die Benutzung von Sprengstoffen ist gesetzlich geregelt, zudem sind sie schwer handhabbar.

Aus dem US-Patent 5,958,299 ist ein Sprengstoff-Simulationsgemisch bekannt, das nicht explosive Bestandteile enthält, wobei die Komponenten so ausgewählt sind, dass die Mischung eine physikalische Form, Dichte, Röntgentransmission und eine effektive Ordnungszahl aufweist, die einem ausgewählten Sprengstoffgemisch entspricht. Mit dem Simulationsgemisch anstelle eines realen Sprengstoffes lässt sich ein Röntgenprüfgerät auf die Detektion des bestimmten Sprengstoffes testen. Als Simulationsgemische sind feste, plastische und gelförmige Zusammensetzungen beschrieben, die sich aus verschiedenen Komponenten aufbauen.Die GB-A-347144 offenbart eine Mischung für ein Spinnbad, die aus Glycerin, Essigsäure und Wasser besteht, wobei der Anteil von Glycerin 50 Gew. %, der Anteil von Essigsäure 25 Gew. %, und der Anteil von Wasser 25 Gew. % beträgt. An Prüfanlagen wird zunehmend die Anforderung gestellt, auch Flüssigkeitssprengstoffe und sogenannte "home made explosives" zu detektieren. Der Erfindung liegt daher die Aufgabe zugrunde, eine derartige Spreng- oder Explosivstoffe simulierende Materialmischung bereitzustellen, die nicht explosiv, unkritisch in der Handhabung und preisgünstig in der Herstellung ist, sowie sich in einem Prüfgerät zur Überprüfung von Gegenständen oder Personen verhält wie der reale Gefahrstoff.

In der DE 10 2011 081 328.4 wird als Simulationsgemisch ein Gemisch aus Glycerin, Natriumoxid (NaOH) und Wasser verwendet, wobei Glycerin und Natriumhydroxid in einem Gewichtsverhältnis Glycerin / Natriumhydroxid zwischen 6,5 und 3,8 vorliegen.

Nach der Erfindung wird zur Simulation von explosiven Flüssigkeiten ein flüssiges Gemisch verwendet, das aus einer Mischung von Glycerin, Essigsäure (CH₃COOH) und Wasser besteht, wobei der Anteil von Glycerin 29,5 Gew.-% - 45,5 Gew.-%, insbesondere etwa 37,5 Gew.-% beträgt. Der Anteil von Essigsäure (CH₃COOH) beträgt 7,625 Gew.-% - 23,625 Gew.-%, insbesondere ca. 15,625 Gew.-%. Die jeweiligen Anteile werden durch Wasser zu 100 Gew.-% ergänzt, wobei der Anteil von Wasser zwischen 41,85 Gew.-% und 51,85 Gew.-%, insbesondere ca. 46,875 Gew.-% beträgt. Der Anteil von Essigsäure lässt sich beispielsweise als 25%ige Essigessenz zugeben. Um 16,625% Essigsäure in der Mischung zu erhalten, werden dann 62,5 Gew.-% der 25%igen Essigessenz zugemischt.

Eine bevorzugte Mischung besteht aus einem Anteil von Glycerin von 37,5 Gew.-%, einem Anteil von 15,625 Gew.-% Essigsäure (CH₃COOH) und 46,875 Gew.-% Wasser.

Für den Test und die Validation wird in einem zu überprüfenden Gegenstand oder an einer Person das flüssige Simulationsgemisch nach der Erfindung angeordnet. Das Simulationsgemisch besteht aus Glycerin, Essigsäure und Wasser.

Die vorstehend beschriebene Grundrezeptur lässt sich unter Beibehaltung der Verhältnisse zwischen Glycerin und Essigsäure durch mehr oder weniger Wasser verdünnen oder verdicken, wobei das Gesamtgemisch flüssig bleibt. Durch die unterschiedlichen Anteile von Wasser lassen sich Simulationsgemische zur Simulation von verschiedenen flüssigen Gefahrstoffen herstellen.

In den Prüfgeräten werden die zu überprüfenden Gegenstände, insbesondere Gepäckstücke, mit elektromagnetischen Strahlen durchleuchtet oder bestrahlt. Die transmittierten oder gestreuten Strahlen werden detektiert und ausgewertet.

Eine bevorzugte Verwendung eines Gemischs nach der Erfindung erfolgt zum Testen und Validieren von Röntgenprüfgeräten zur Überprüfung von Personen oder Gegenständen, insbesondere von Gepäckstücken, wie sie in der Beschreibungseinleitung als bekannt beschrieben werden.

Ebenso kann das Simulationsgemisch nach der Erfindung verwendet werden, um Prüfgeräte zu testen und Validieren, in denen elektromagnetische mm-Wellen zur Überprüfung von Personen oder Gegenständen verwendet werden.

## Patentansprüche

1. Flüssiges Gemisch zum Testen und Validieren von Prüfgeräten zur Überprüfung von Gegenständen oder Personen, das Glycerin enthält und aus einer Mischung von Glycerin, Essigsäure und Wasser besteht, **dadurch gekennzeichnet, dass** der Anteil von Glycerin 29,5 Gew.-%-45,5 Gew.-%, insbesondere etwa 37,5 Gew.-%, der Anteil von Essigsäure 7,625 Gew.-% - 23,625 Gew.-%, insbesondere ca. 15,625 Gew.-%, und der Anteil von Wasser 41,85 Gew.-% - 51,85 Gew.-%, insbesondere ca. 46,875 Gew.-% beträgt.

## Claims

1. Liquid mixture for testing and validating screening devices for inspecting objects or persons which contains glycerol and consists of a mixture of glycerol, acetic acid and water, **characterized in that** the proportion of glycerol is 29.5 wt%-45.5 wt%, especially about 37.5 wt%, the proportion of acetic acid is 7.625 wt%-23.625 wt%, especially *circa* 15.625 wt%, and the proportion of water is 41.85 wt%-51.85 wt%, especially *circa* 46.875 wt%.

## Revendications

1. Mélange liquide pour tester et valider des appareils de contrôle pour le contrôle d'objets ou de personnes, qui contient de la glycérine et est constitué par un mélange de glycérine, d'acide acétique et d'eau, **caractérisé en ce que** la proportion de glycérine est de 29,5 % en poids à 45,5 % en poids, notamment d'environ 37,5 % en poids, la proportion d'acide acétique est de 7,625 % en poids à 23,625 % en poids, notamment d'environ 15,625 % en poids, et la proportion d'eau est de 41,85 % en poids à 51,85 % en poids, notamment d'environ 46,875 % en poids.
